# EUROPEAN PATENT SPECIFICATION

(11) **EP 0 561 866 B1**
(45) Date of publication and mention of the grant of the patent: **30.08.1995**
(21) Application number: 92900368.9
(22) Date of filing: 09.12.1991
(51) Int. Cl.: A61K 31/195, A61K 9/14

(54) **LYOPHILIZED AMINO ACID COMPOSITIONS CONTAINING GLUTAMINE**
GLUTAMINHALTIGE LYOPHILISIERTE AMINOKARBONSÄURENZUSAMMENSETZUNGEN
COMPOSITIONS LYOPHILISEES D'ACIDES AMINES CONTENANT DE LA GLUTAMINE

(30) Priority: 11.12.1990 IT 2234290
(43) Date of publication of application: 29.09.1993
(73) Proprietor: Dr. A. TORRE FARMACEUTICI S.R.L., I-20134 Milano (IT)
(72) Inventor: TORRE, Alberto, I-20134 Milano (IT)
(74) Representative: Minoja, Fabrizio
(86) International application number: EP9102352
(87) International publication number: WO9210175

(56) References cited:
- WO-A-87/01589
- JOURNAL OF PARENTERAL AND ENTERAL NUTRITION, vol. 14, no. 4, Suppl., 1990, 137S-146S
- JOURNAL OF PARENTERAL AND ENTERAL NUTRITION, vol. 14, no. 4, 1990, 344-352
- JOURNAL OF PARENTERAL AND ENTERAL NUTRITION, vol. 14, no. 4, Suppl., 1990, 100S-105S

## Description

The present invention refers to amino acids compositions for the parenteral administration containing glutamine, in the form of sterile powder to be dissolved in sterile injectable solutions before use and to a method for the preparation thereof comprising solubilization, filtration and lyophilization.

Glutamine is a non-essential amino acid, since it may be synthesized in the body from other amino acids and precursors in an amount adequate to the physiological requirement.

It is the most abundant amino acid in the organism since it constitutes about 50% of the free intracellular amino acid pool of the muscular tissue.

The glutamine concentration, however, decreases rapidly when sepsis, trauma or other serious diseases occur, since the requirement is not sufficiently supported by the synthesis capacity of the organism. This decrease in glutamine levels causes a reduction of the protein synthesis, a decrease in the immune defenses and the atrophy of the intestinal mucosa.

Meister (Physiol. Rev. 36:103-127, 1956) has already suggested the dietetic supplementation with glutamine to satisfy the increased metabolism of some cells or tissues.

Kapadia et al. (JPEN 6:583-589, 1985) reported that the glutamine infusion in a slightly catabolic model was able to preserve the intracellular levels of this amino acid.

Albers et al. (Akt. Ernährungsmed 9:147-149, 1984) were successful in sustaining the glutamine level in intra- and extra-cellular pools of muscular tissue by infusion of the dipeptide alanylglutamine in rats subjected to total parenteral feeding.

US 4,857,555 discloses the use of glutamine in the parenteral nutrition to prevent the catabolism of the muscular tissue, the atrophy of intestinal villi and of other dysfunctions. Even though many proposals for the preparation of glutamine formulations, including lyophilization of the amino acid, are generically included in the specification of US 4,857,555 no actual, practically applicable embodiment is given. In fact, in the used pharmacological tests, glutamine has always been administered in form of aqueous solution sterilized through a 0.22 »m membrane and mixed immediately before the use with other solutions for parenteral nutrition. The glutamine solution was preserved for 24 hours in the refrigerator so that the problem of the glutamine stability in the formulations for parenteral nutrition was not even addressed.

In fact, at present, infusion solutions of amino acids containing also glutamine are not available on the market, since this amino acid is very little soluble and is unstable in aqueous solution and it undergoes cyclization forming NH₃ and pyroglutamic acid. 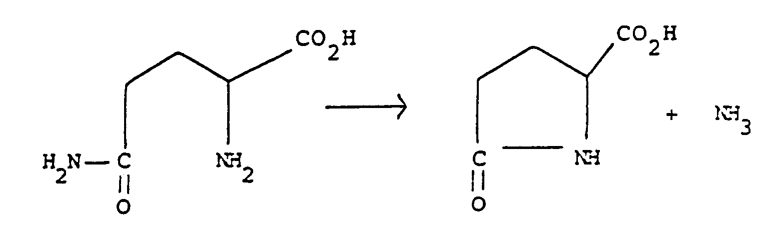
This reaction occurs slowly at room temperature, but it is dramatically accelerated at sterilization temperature in autoclave. In order to overcome this problem, the use has been proposed, of stable derivatives in aqueous solution such as acetylglutamine and dipeptides, for instance alanylglutamine and glycylglutamine, which can be added to the infusional solutions containing the other amino acids.

But this solution has some drawbacks: acetylglutamine has a low bioavailability, whereas the dipeptides supply, besides glutamine, equimolar amounts of other amino acids which can be undesirable, such as glycine and which, anyhow, in view of the high dosage used, make the overall amino acidic pattern of the mixture unbalanced.

As another solution to this problem, the use of sterile lyophilized glutamine to be rehydrated or to be mixed with other powder components immediately before use was proposed. This solution is not of industrial application for the following reasons:
1) glutamine solubility is about 2.5%, therefore the lyophilization process is very expensive;
2) lyophilized glutamine is a very soft powder with very low density and poor flowability, therefore the use of automatic packaging machines is impaired;
3) lyophilized glutamine can be rehydrated only at a concentration lower than 2%, determining a water contribution non compatible with normal parenteral nutrition schemes; moreover, even so diluted solutions have a microparticle content which is pharmaceutically unacceptable.

The present invention concerns the preparation of different injectable mixtures of amino acids and glutamine in lyophilized form to be dissolved in water before use or in the usual infusional solutions such as 5%, 10%, 30%, 50% glucose solutions. The invention provides the remarkable advantage of preventing the phenomenon of degradation of glutamine, thus allowing to preserve the amino acid compositions for a long time and making glutamine bioavailable immediately before use.

The pharmaceutical composition of the invention enables the preparation of formulations having a high glutamine content of from 20 to 50% by weight in the presence of a generally balanced amino acidic content, avoiding the use of excipients, preservatives and stabilizers.

The compositions of the invention have a density ≧ 0.3 g/ml, a solubility of 10% in water and at least of 5% in glucose solutions with glucose concentrations ranging from 5 to 50%. The reconstituted solutions have a particulate count lower than these limits:
1.000 particles ≧ 2 »m
100 particles ≧ 5 »m
50 particles ≧ 10 »m
4 particles ≧ 20 »m.

The preparation process consists in the solubilization in water of the various amino acids present in the formulation and of glutamine, at a concentration from 10 to 20% w/v.

The solution is then filtered through sterilizing membrane, having pore size of 0.22 »m, at room temperature, and then it is freeze-dryed: the lyophilized product is distributed in the previously sterilized primary containers. All the operations are carried out in strictly aseptic conditions.

The amino acids contained in the solution act as glutamine lyophilization support and the final product, due to its density, flowability and physical characteristics, is suitable for industrial packaging. The rehydratation of the lyophilized product is immediate and complete. Injectable water or 5 to 50% glucose solution can be used as solvent. In spite of the amino acid content, ranging from 10% to 5%, microparticle count in the solution is far below the pharmaceutically acceptable limits.

According to the present invention, the lyophilized product allows to obtain solutions with a glutamine concentration higher than the one obtained with lyophilized glutamine only.

The compositions of the invention may be used in human therapy also in combination with glucides, lipids, vitamins, mineral salts according to the parenteral nutrition schemes. The percentages of amino acids and glutamine will be dependent on the patient's needs and on the physician's decision.

The invention is illustrated further by the following formulations exemplified in the table reporting the amino acid composition as percent by weight. It is evident that other amino acids can also be used according to specific nutritional needs.

**TABLE 1**

| | I | II | III | IV |
|---|---|---|---|---|
| Isoleucine | 8,5 | 8,5 | 6,2 | 6,2 |
| Leucine | 13,0 | 13,0 | 8,6 | 8,6 |
| Lysine | 7,0 | 7,0 | 7,9 | 7,9 |
| (Lysine acetate) | (9,9) | (9,9) | (11,2) | (11,2) |
| Methionine | 3,0 | 3,0 | 4,0 | 4,0 |
| Phenylalanine | 3,0 | 3,0 | 4,9 | 4,9 |
| Treonine | 5,0 | 5,0 | 5,0 | 5,0 |
| Tryptophan | 1,0 | 1,0 | 1,5 | 1,5 |
| Valine | 9,0 | 9,0 | 7,2 | 7,2 |
| Total Ess. Am. | 49,5 | 49,5 | 45,3 | 45,3 |
| Arginine | 2,5 | 1,0 | 2,1 | |
| Hystidine | 2,5 | 1,5 | 2,4 | 1,0 |
| Alanine | 7,5 | 6,5 | 4,0 | 1,5 |
| Glycine | 4,5 | 2,5 | 2,2 | |
| Proline | 8,5 | 6,0 | 2,0 | 1,2 |
| Serine | 5,0 | 3,0 | 2,0 | 1,0 |
| Glutamine | 20,0 | 30,0 | 40,0 | 50,0 |

Three batches corresponding to the formulation n. III were industrially produced.

The lyophilized product was distributed under nitrogen in doses of 25 g in 500 ml infusion bottles.

The solution was prepared by transferring aseptically 250 ml of water for injectable preparations or 500 ml of 5%, 10%, 20%, 30%, 50% glucose solutions in the lyophilized bottle.

The solution was sterile and apyrogenic with microparticle count within the limits stated by the Official Pharmacopoeia.
The stability of the compositions of the invention was evaluated according to the following tests:
the amino acids assay by means of Amino acid Analyzer;
the glutamine titer by means of Amino acid Analyzer;
the pyroglutamic acid titer by HPLC.
The product remained unchanged at room temperature for more than 12 months.
The reconstituted solutions were stable for more than 15 hours at room temperature and for more than 48 hours at 4°C.

## Claims

1. Amino acids compositions for parenteral administration, containing glutamine in amounts from 20 to 50% by weight in form of sterile lyophilized powder, to be dissolved in sterile injectable solutions before use, said powder having a density ≧ 0.3 g/ml, a solubility of 10% in water and at least of 5% in glucose solutions with glucose concentrations ranging from 5 to 50%, stability longer than 12 months at room temperature in the dry state and longer than 15 hours at room temperature or longer than 48 hours at 4°C, in reconstituted solution, said solution having a microparticle count lower than these limits:
1.000 particles ≧ 2 »m
100 particles ≧ 5 »m
50 particles ≧ 10 »m
4 particles ≧ 20 »m.

2. A process for the preparation of lyophilized compositions of amino acids of claim 1 comprising:
a) solubilization of the amino acids and glutamine in water for injectable preparations;
b) filtration of the obtained solution through sterile membrane at room temperature;
c) freeze-drying of the filtrate;
d) distribution in sterile containers under nitrogen.

## Patentansprüche

1. Aminosäurezusammensetzungen für die parenterale Verabreichung, enthaltend Glutamin in Mengen von 20 bis 50 Gew.-% in Form eines sterilen lyophilisierten Pulvers zur Auflösung in sterilen injizierbaren Lösungen vor Gebrauch, wobei das Pulver eine Dichte ≧ 0,3 g/ml, eine Löslichkeit von 10% in Wasser und mindestens 5% in Glucoselösungen mit Glucosekonzentrationen im Bereich von 5 bis 50%, eine Stabilität von mehr als 12 Monaten bei Raumtemperatur in trockenem Zustand und mehr als 15 Stunden bei Raumtemperatur oder mehr als 48 Stunden bei 4°C in der rekonstituierten Lösung besitzt, wobei die Lösung eine Mikroteilchenzahl niedriger als die angegebenen Grenzen besitzt:
1000 Teilchen ≧ 2 »m
100 Teilchen ≧ 5 »m
50 Teilchen ≧ 10 »m
4 Teilchen ≧ 20 »m.

2. Verfahren zur Herstellung von lyophilisierten Aminosäurezusammensetzungen gemäß Anspruch 1, dadurch **gekennzeichnet**, daß man
a) die Aminosäuren und Glutamin in Wasser für Injektionspräparate solubilisiert;
b) die so erhaltene Lösung durch eine sterile Membran bei Raumtemperatur filtriert;
c) das Filtrat gefriertrocknet;
d) das Präparat in sterile Behältnisse unter Stickstoff verteilt.

## Revendications

1. Compositions d'acides aminés pour l'administration parentérale, contenant de la glutamine en des quantités allant de 20 à 50 % en poids, sous la forme de poudre stérile lyophilisée, à dissoudre dans des solutions stériles injectables avant l'utilisation, ladite poudre ayant une masse volumique ≧ 0,3 g/ml, une solubilité de 10 % dans l'eau et au moins de 5 % dans des solutions de glucose, les concentrations de glucose étant de 5 à 50 %, une stabilité supérieure à 12 mois à température ambiante à l'état sec, et supérieure à 15 heures à température ambiante ou supérieure à 48 heures à 4°C en solution reconstituée, ladite solution contenant un nombre de microparticules inférieur à ces limites :
1000 particules ≧ 2 »m
100 particules ≧ 5 »m
50 particules ≧ 10 »m
4 particules ≧ 20 »m.

2. Procédé pour la préparation de compositions lyophilisées d'acides aminés selon la revendication 1, comprenant :
a) la solubilisation des acides aminés et de la glutamine dans de l'eau pour des préparations injectables ;
b) la filtration de la solution obtenue à travers une membrane stérile à température ambiante ;
c) la lyophilisation du filtrat ;
d) la répartition dans des récipients stériles sous azote.
